# FASCICULE DE BREVET EUROPEEN

(11) **EP 1 169 300 B1**
(45) Date de publication et mention de la délivrance du brevet: **28.05.2003**
(21) Numéro de dépôt: 00912739.0
(22) Date de dépôt: 24.03.2000
(51) Int. Cl.: C07C 235/16, A61K 7/06

(54) **COMPOSES DERIVES D'ESTERS D'ACIDE BENZOIQUE, COMPOSITION LES COMPRENANT ET UTILISATION**
BENZOESÄUREESTERDERIVATE, DIESE ENTHALTENDE ZUSAMMENSTELLUNGEN UND DEREN ANWENDUNG
COMPOUNDS DERIVED FROM BENZOIC ACID ESTERS, COMPOSITION CONTAINING SAID COMPOUNDS AND USE THEREOF

(30) Priorité: 01.04.1999 FR 9904104
(43) Date de publication de la demande: 09.01.2002
(73) Titulaire: L'OREAL, 75008 Paris (FR)
(72) Inventeur: GALEY, Jean-Baptiste, F-93600 Aulnay (FR); DALKO, Maria, F-91190 Gif sur Yvette (FR); BRETON, Lionel, F-78000 Versailles (FR)
(74) Mandataire: Dodin, Catherine
(86) Numéro de dépôt international: FR0000752
(87) Numéro de publication internationale: WO00059866

(56) Documents cités:
- WO-A-97/32562
- KING F D: "BIOISOSTERES, CONFORMATIONAL RESTRICTION, AND PRO-DRUGS - CASE HISTORY: AN EXAMPLE OF A CONFORMATIONAL RESTRICTION APPROACH" GB,CAMBRIDGE, RSC,1994, page 206-209 XP002033086 ISBN: 0-85186-494-5
- THOMAS D. AICHER ET AL.: "(R)-3,3,3-trifluoro-2-hdroxy-2-methylprop ionamides are orally active inhibitors of pyruvate dehydrogenase kinase" JOURNAL OF MEDICINAL CHEMISTRY., vol. 42, no. 15, juillet 1999 (1999-07), pages 2741-2746, XP002122777 AMERICAN CHEMICAL SOCIETY. WASHINGTON., US ISSN: 0022-2623

## Description

La présente invention a trait à des dérivés d'esters d'acide benzoïque, à leur utilisation notamment en cosmétique, ainsi qu'aux compositions les comprenant.

Chez l'être humain, la croissance des cheveux et leur renouvellement sont principalement déterminés par l'activité des follicules pileux et de leur environnement dermo-épidermique. Leur activité est cyclique et comporte essentiellement trois phases, à savoir la phase anagène, la phase catagène et la phase télogène.
A la phase anagène active ou phase de croissance, qui dure plusieurs années et au cours de laquelle les cheveux s'allongent, succède une phase catagène très courte et transitoire qui dure quelques semaines, puis une phase de repos, appelée phase télogène, qui dure quelques mois.
A la fin de la période de repos, les cheveux tombent et un autre cycle recommence. La chevelure se renouvelle donc en permanence, et sur les 150000 cheveux environ que comporte une chevelure, à chaque instant, 10% d'entre eux environ sont au repos et seront donc remplacés en quelques mois.

Dans un nombre important de cas, la chute précoce des cheveux survient chez des sujets prédisposés génétiquement et elle atteint notamment les hommes. Il s'agit plus particulièrement de l'alopécie androgénétique ou androgénique ou encore androgéno-génétique.
Cette alopécie est essentiellement due à une perturbation du renouvellement capillaire qui entraîne, dans un premier temps, l'accélération de la fréquence des cycles aux dépens de la qualité des cheveux puis de leur quantité. Il se produit un appauvrissement progressif de la chevelure par régression des cheveux dits "terminaux" au stade de duvets. Des zones sont touchées préférentiellement; notamment chez l'homme, les golfes temporaux ou frontaux ainsi que la partie supérieure de l'occipital, tandis que chez les femmes on constate plutôt une alopécie diffuse du vertex.

On recherche depuis de nombreuses années, notamment dans l'industrie cosmétique, des substances permettant de supprimer ou de réduire l'effet de l'alopécie, et notamment d'induire ou de stimuler la croissance des cheveux et/ou des poils, voire de diminuer leur chute. Dans cette optique, on a certes déjà proposé un grand nombre de composés actifs très divers, comme par exemple le 2,4-diamino 6-piperidino pyrimidine 3-oxyde ou "Minoxidil" décrit dans US 4 596 812 ou encore ses nombreux dérivés comme ceux décrits par exemple dans les demandes de brevet EP 353123, EP 356271, EP 408442, EP 522964, EP 420707, EP 459890 et EP 519819. On peut encore citer le 6-amino 1,2-dihydro 1-hydroxy 2-imino 4-pipéridino pyrimidine et ses dérivés, qui sont décrits plus particulièrement dans le brevet US-A- 4 139 619.

WO-A-97/32562 décrit des composés de la famille des N-aryl--2-hydroxyalkylamides utiles pour induire et/ou stimuler la croissance des cheveux et/ou freiner leur chute.

Il reste, d'une manière générale, qu'il serait intéressant et utile de pouvoir disposer de composés actifs autres que ceux déjà connus, de préférence potentiellement plus actifs et/ou moins toxiques, et susceptibles d'être employés dans le domaine cosmétique.

Or, après de nombreuses recherches, la demanderesse vient de mettre en évidence que des composés, dérivés d'esters d'acide benzoïque, présentaient des propriétés remarquables susceptibles de justifier leur utilisation pour diminuer et/ou freiner la chute des cheveux et/ou des poils. Ces composés pourraient éventuellement également être susceptibles d'induire et/ou stimuler leur croissance.

Ainsi, l'invention a pour objet des composés répondant à la formule (I) suivante : 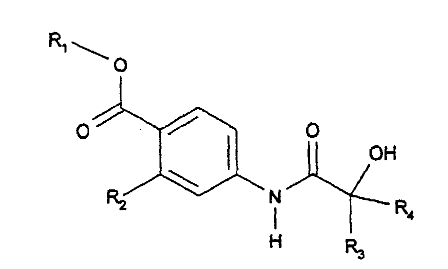 dans laquelle :
* R₁ représente :
   - un groupement alkyle aliphatique en C₁-C₁₂, linéaire ou ramifié, éventuellement substitué par un ou plusieurs groupements choisis parmi OH, NH₂, SH; CONHR⁵, COOR⁵, OR⁵, SR⁵, SiOR⁵, NHR⁵ dans lesquels R⁵ est un radical alkyle linéaire ou ramifié en C₁-C₄; CN, CF₃, halogène, ou par un ou plusieurs cycles en C₃-C₆ aliphatiques ou aromatiques, éventuellement hétérocycliques;
   - un groupement aryle, éventuellement substitué par un ou plusieurs groupements OH, NH₂, SH, COOH; CONHR⁶, COOR⁶, OR⁶, SR⁶, NHR⁶ dans lesquels R⁶ est un radical alkyle linéaire ou ramifié en C₁-C₁₂; CN, CF₃, halogène ou par un ou plusieurs cycles en C₃-C₆ aliphatiques ou aromatiques, éventuellement hétérocycliques; ou
   - un polymère ou copolymère naturel ou synthétique portant une ou plusieurs combinaisons des groupements suivants: hydroxyle, carboxylate, amine primaire, amine secondaire, amine tertiaire, thiol, aldéhyde.
* R₂ représente un atome d'hydrogène, d'halogène, un radical CN, CF₃, OH, OCF₃, COOH, R⁷, OR⁷ ou OCOR⁷ dans lesquels R⁷ désigne un radical alkyle aliphatique en C₁-C₄ linéaire ou ramifié.
* R₃ représente un radical alkyle linéaire ou ramifié en C₁-C₄ mono- ou poly-halogéné, ou un radical aryle, éventuellement substitué par un ou plusieurs groupements choisis parmi OH, NH₂, SH, CN, CF₃, halogène, COOH, CONHR⁸, COOR⁸, OR⁸, SR⁸, NHR⁸ dans lesquels R⁸ est un radical alkyle en C₁-C₁₂, linéaire ou ramifié.
* R₄ représente un radical alkyle en C₁-C₄ linéaire ou ramifié, éventuellement substitué par un atome d'halogène, ou représente un radical CF₃.

L'invention a également pour objet une composition, notamment cosmétique, comprenant, dans un milieu physiologiquement acceptable, au moins un composé tel que défini ci-dessus.
L'invention a encore pour objet l'utilisation, dans une composition notamment cosmétique, ou pour la préparation d'une composition physiologiquement acceptable, d'au moins un composé répondant à la formule (I) pour diminuer et/ou freiner la chute des cheveux et/ou des poils, voire induire et/ou stimuler leur croissance.

Un des avantages de l'invention réside dans le fait que les composés selon l'invention sont dénués d'effets secondaires, dans la mesure où ils se métabolisent en un acide dénué d'effet anti-androgène.

Les composés selon l'invention sont représentés par la formule (I) suivante : 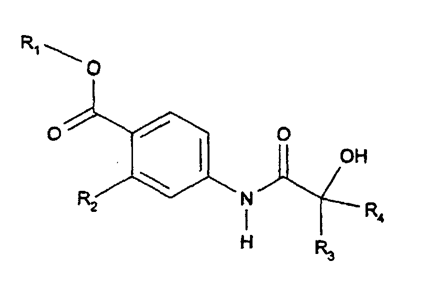 dans laquelle :
* R₁ représente :
   - un groupement alkyle aliphatique en C₁-C₁₂ , linéaire ou ramifié, éventuellement substitué par un ou plusieurs groupements choisis parmi OH, NH₂, SH; CONHR⁵, COOR⁵, OR⁵, SR⁵, SiOR⁵, NHR⁵ dans lesquels R⁵ est un radical alkyle linéaire ou ramifié en C₁-C₄; CN, CF₃, halogène, ou par un ou plusieurs cycles en C₃-C₆ aliphatiques ou aromatiques, éventuellement hétérocycliques;
   - un groupement aryle, éventuellement substitué par un ou plusieurs groupements OH, NH₂, SH, COOH; CONHR⁶, COOR⁶, OR⁶, SR⁶, NHR⁶ dans lesquels R⁶ est un radical alkyle linéaire ou ramifié en C₁-C₁₂; CN, CF₃, halogène ou par un ou plusieurs cycles en C₃-C₆ aliphatiques ou aromatiques, éventuellement hétérocycliques; ou
   - un polymère ou copolymère naturel ou synthétique portant une ou plusieurs combinaisons des groupements suivants: hydroxyle, carboxylate, amine primaire, amine secondaire, amine tertiaire, thiol, aldéhyde.
* R₂ représente un atome d'hydrogène, d'halogène, un radical CN, CF₃, OH, OCF₃, COOH, R⁷, OR⁷ ou OCOR⁷ dans lesquels R⁷ désigne un radical alkyle aliphatique en C₁-C₄ linéaire ou ramifié.
* R₃ représente un radical alkyle linéaire ou ramifié en C₁-C₄ mono ou poly halogéné, ou un radical aryle, éventuellement substitué par un ou plusieurs groupements choisis parmi OH, NH₂, SH, CN, CF₃, halogène, COOH, CONHR⁸, COOR⁸, OR⁸, SR⁸, NHR⁸ dans lesquels R⁸ est un radical alkyle en C₁-C₁₂, linéaire ou ramifié.
* R₄ représente un radical alkyle en C₁-C₄ linéaire ou ramifié, éventuellement substitué par un atome d'halogène, ou représente un radical CF₃.

Parmi les polymères naturels susceptibles d'être représenter par R₁, on peut citer les polymères naturels modifiés tels que les dérivés éthers ou esters de cellulose, les polysaccharides, les oligosaccharides et les glycosaminoglucanes.

De préférence, R₁ représente un groupement aryle éventuellement substitué, et notamment un groupement phényle ou benzyle.
De préférence, R₂ représente un atome d'hydrogène, un atome d'halogène et notamment un atome de chlore, ou un radical hydroxy.
De préférence, R₃ représente un radical alkyle halogéné, notamment fluoré, ou un radical phényle. En particulier, R₃ peut représenter le radical CF₃.
De préférence, R₄ représente un radical méthyle.

Parmi les composés de formule (I), on peut plus particulièrement citer :
- l'ester 4-(O-methyloxime)-phénylique de l'acide 2-hydroxy-4-(3,3,3-trifluoro-2-hydroxy-2-methyl propionylamino)-benzoïque;
- l'ester phénylique de l'acide 2-hydroxy-4-(3,3,3-trifluoro-2-hydroxy-2-methyl propionylamino)-benzoïque;
- l'ester méthylique de l'acide 4-(3,3,3-trifluoro-2-hydroxy-2-methyl propionylamino)-benzoïque;
- l'ester para-tolyl de l'acide 2-hydroxy-4-(3,3,3-trifluoro-2-hydroxy-2-methyl propionylamino)-benzoïque;
- l'ester phenylique de l'acide 2-hydroxy-4-(2-phenyl-2-hydroxy propionylamino)-benzoïque;
- l'ester méthylique de l'acide 2-méthoxy-4-(3,3,3-trifluoro-2-hydroxy-2-methyl propionylamino)-benzoïque;
- l'ester phénylique de l'acide 2-chloro-4-(3,3,3-trifluoro-2-hydroxy-2-methyl propionylamino)-benzoïque;
- l'ester méthylique de l'acide 2-ethyl-4-(3,3,3-trifluoro-2-hydroxy-2-methyl propionylamino)-benzoïque;
- l'ester 3,5-bis-trifluorométhyl-benzylique de l'acide 2-hydroxy-4-(3,3,3-trifluoro-2-hydroxy-2-methyl propionylamino)-benzoïque;
- l'ester tert-butylique de l'acide 2-hydroxy-4-(3,3,3-trifluoro-2-hydroxy-2-methyl propionylamino)-benzoïque;
- l'ester 3-morpholino-propylique de l'acide 2-hydroxy-4-(3,3,3-trifluoro-2-hydroxy-2-methyl propionylamino)-benzoïque;
- l'ester 4-octyl-phénylique de l'acide 2-hydroxy-4-(3,3,3-trifluoro-2-hydroxy-2-methyl propionylamino)-benzoïque; et
- l'ester méthylique de l'acide 2-hydroxy-4-(3,3,3-trifluoro-2-hydroxy-2-methyl propionylamino)-benzoïque.

Les composés de formule (I) peuvent être utilisés seuls ou en mélange.

La quantité de composé à utiliser dans les compositions selon l'invention peut être aisément déterminée par l'homme du métier, en fonction de la nature du composé utilisé, de la personne à traiter et/ou de l'effet recherché. D'une manière générale, cette quantité peut être comprise entre 0,0001 et 30% en poids par rapport au poids total de la composition, notamment entre 0,01 et 10% en poids, et de préférence entre 0,1 et 5% en poids.

Ainsi, les composés de formule (I) selon l'invention peuvent être notamment employés dans une composition ou pour la préparation d'une composition qui comprend par ailleurs un milieu physiologiquement acceptable.
Cette composition peut se présenter sous la forme d'une composition cosmétique qui comprend donc un milieu cosmétiquement acceptable.
Le milieu physiologiquement acceptable dans lequel les composés selon l'invention peuvent être employés est aisément déterminable par l'homme du métier.
D'une manière générale, il peut être anhydre ou aqueux.
On entend par milieu anhydre, un milieu solvant contenant moins de 1% en poids d'eau. Ce milieu peut être constitué d'un solvant organique ou d'un mélange de solvants organiques choisis plus particulièrement parmi les alcools en C₁-C₄ comme l'éthanol; les alkylèneglycols comme le propylèneglycol; les alkyléthers d'alkylèneglycols ou de dialkylèneglycols, dont les- radicaux alkyle ou alkylène contiennent de 1 à 4 atomes de carbone.
On entend par milieu aqueux, un milieu constitué par de l'eau ou par un mélange d'eau et d'un autre solvant physiologiquement acceptable, choisi notamment parmi les solvants organiques cités ci-dessus. Dans ce dernier cas, ces autres solvants peuvent représenter environ 5 à 95% en poids de la composition.

Il est possible d'ajouter dans la composition, en association avec les composés selon l'invention, un ou plusieurs composés améliorant encore l'activité sur la repousse et/ou sur le freinage de la chute des cheveux, et ayant déjà été décrits pour cette activité.
On peut plus particulièrement citer à titre non limitatif :
- les esters d'acide nicotinique, dont notamment le nicotinate de tocophérol, le nicotinate de benzyle et les nicotinates d'alkyles en C₁-C₆ comme les nicotinates de méthyle ou d'hexyle ;
- les dérivés de pyrimidine, comme le 2,4-diamino 6-piperidinopyrimidine 3- oxyde ou "Minoxidil" décrit dans les brevets US 4 139 619 et US 4 596 812 ;
- les agents favorisant la repousse des cheveux comme ceux décrits dans la demande de brevet EP0648488 ;
- les agents antibactériens tels que les macrolides, les pyranosides et les tétracyclines, et notamment l'érythromycine ;
- les agents antagonistes de calcium, comme la cinnarizine, le diltiazem, la nimodipine et la nifedipine ;
- des hormones telles que l'estriol et ses analogues, ou la thyroxine et ses sels ;
- des agents anti-inflammatoires stéroïdiens ou non stéroidiens, tels que les corticostéroïdes (par exemple : l'hydrocortisone) ;
- des agents antiandrogènes, tels que l'oxendolone, la spironolactone, le diéthylstilbestrol et la flutamide ;
- des inhibiteurs stéroïdiens ou non stéroïdiens des 5-α-réductases tels que le finastéride
- des agonistes potassiques tels que la cromakalim et le nicorandil.
- des agonistes des récepteurs de RXR des rétinoïdes et des antagonistes des rétinoïdes ;
- des capteurs de radicaux OH, tels que le diméthylsulfoxyde ;
- des peptides comme par exemple le tripeptide Lys-Pro-Val, et plus généralement l'α-MSH et ses dérivés.

On peut ajouter à cette liste, les composés suivants :
- le diazoxyde, la spiroxasone,
- des phospholipides comme la lécithine,
- les acides linoléique et linolénique,
- l'acide salicylique et ses dérivés décrits dans le brevet français FR 2 581 542, comme les dérivés de l'acide salicylique porteurs d'un groupement alcanoyle ayant de 2 à 12 atomes de carbone en position 5 du cycle benzénique,
- des acides hydroxycarboxyliques ou cétocarboxyliques et leurs esters,
- des lactones et leurs sels correspondants,
- l'anthraline, des caroténoides,
- les acides eicosatétrayénoïque et eicosatriyénoïque ou leurs esters et amides,
- la vitamine D et ses dérivés,
- des extraits d'origine végétale ou bactérienne.

D'autre part, on peut ajouter au milieu physiologiquement acceptable, des adjuvants habituellement utilisés dans le domaine d'application considéré, notamment dans le domaine cosmétique, tels que des agents tensioactifs, des agents épaississants ou gélifiants, des agents cosmétiques, des agents conservateurs, des agents alcalinisants ou acidifiants bien connus dans l'état de la technique.
La nature et la quantité de ces adjuvants peuvent être choisies par l'homme du métier, sur la base de ses connaissances générales, de manière à obtenir la forme de présentation désirée pour la composition. En tout état de cause, l'homme du métier veillera à choisir tous les éventuels composés complémentaires et/ou leur quantité, de manière telle que les propriétés avantageuses de la composition selon l'invention ne soient pas, ou substantiellement pas, altérées par l'adjonction envisagée.

Les compositions selon l'invention peuvent se présenter sous la forme de lotion plus ou moins épaissie, de gel, d'émulsion, ou de crème. L'utilisation peut éventuellement se faire sous une forme pressurisée en aérosol ou vaporisée à partir d'un flacon pompe.
Les compositions peuvent également se présenter sous forme liposomée, telle que notamment décrite dans la demande de brevet WO 94/22468. Dans ce cas, le composé encapsulé dans les liposomes peut être délivré sélectivement au niveau du follicule pileux.

Les compositions selon l'invention peuvent être appliquées sur les zones alopéciques du cuir chevelu et/ou des cheveux et/ou des poils d'un individu, puis être éventuellement laissées en contact plusieurs heures et être éventuellement rincées.
On peut, par exemple, appliquer sur les cheveux ou le cuir chevelu la composition comprenant une quantité efficace de composé selon l'invention, le soir; garder celle-ci au contact toute la nuit et éventuellement effectuer un shampooing le matin. Ces applications peuvent être renouvelées quotidiennement pendant un ou plusieurs mois selon les individus.

Ainsi, la présente invention à également pour objet un procédé de traitement cosmétique des cheveux, des poils et/ou du cuir chevelu, dans lequel on applique sur les cheveux, les poils et/ou le cuir chevelu, une composition cosmétique comprenant une quantité efficace d'au moins un composé de formule (I), à laisser celle-ci en contact avec les cheveux et/ou le cuir chevelu et/ou les poils, et éventuellement à rincer.
Le procédé de traitement présente les caractéristiques d'un procédé cosmétique dans la mesure où il permet d'améliorer l'esthétique des cheveux en leur donnant une plus grande vigueur et un aspect amélioré.

L'invention est illustrée plus en détail dans les exemples suivants.

### Exemple 1 : préparation de l'ester phénylique de l'acide 2-hydroxy-4-(3,3,3-trifluoro-2-hydroxy-2-methyl propionylamino)-benzoïque.

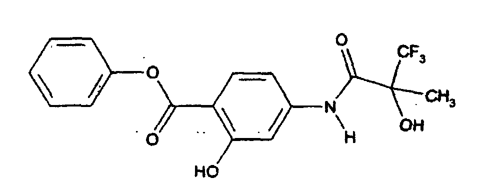

On solubilise 2,1 g d'acide 3,3,3-trifluoro-2-hydroxy-2-methylpropanoïque dans 20ml de N,N-dimethylacétamide anhydre. Le milieu est refroidi à -20°C, puis on ajoute 1 ml de chlorure de thionyle goutte à goutte. Le milieu réactionnel est agité pendant 1 heure à -20°C, puis on ajoute 2,0 g de 4-amino salicylate de phényle.
On laisse la température remonter à environ 25°C et on agite pendant une nuit (12 heures). Le mélange est versé dans 100 ml d'eau glacée et extrait 3 fois avec 50 ml de dichlorométhane.
La phase organique est lavée à l'eau puis séchée sur Na₂SO₄ et évaporée à sec.

On obtient une huile jaune que l'on purifie par chromatographie sur colonne de silice (éluant : dichlorométhane). L'huile obtenue après évaporation à sec est reprise dans 20 ml d'un mélange heptane / toluène 1:1.
On obtient un solide blanc qui est recristallisé dans 10 ml de toluène et séché sous vide.
On obtient 1,35 g d'ester phénylique de l'acide 2-hydroxy-4-(3,3,3-trifluoro-2-hydroxy-2-methyl propionylamino)-benzoïque (rendement = 50 %).
C₁₇ H₁₄ F₃ N O₅ PM=369
- Spectre RMN : ¹H (400 MHz) dans CDCl₃ : conforme à la structure attendue

### - Analyse élémentaire

| | C | H | N | F |
|---|---|---|---|---|
| % calculé | 55,28 | 3,79 | 3,79 | 15,45 |
| % trouvé | 55,23 | 3,73 | 3,85 | 15,71 |

### Exemple 2

De la même manière qu'à l'exemple 1, on prépare les composés suivants :
(a) l'ester méthylique de l'acide 4-(3,3,3-trifluoro-2-hydroxy-2-methyl propionylamino)-benzoïque de formule 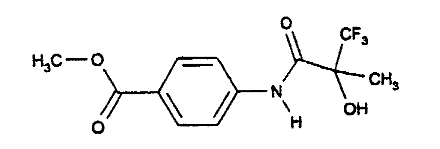 Spectre RMN : ¹H (500 MHz) dans DMSO : conforme à la structure attendue
(b) l'ester para-tolyl de l'acide 2-hydroxy-4-(3,3,3-trifluoro-2-hydroxy-2-methyl propionylamino)-benzoïque de formule 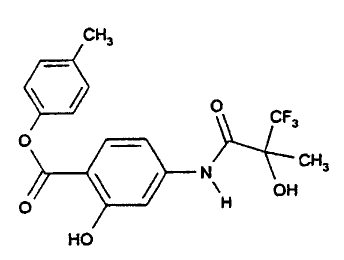 Spectre RMN : ¹H (500 MHz) dans DMSO : conforme à la structure attendue
(c) l'ester phénylique de l'acide 2-hydroxy-4-(2-phenyl-2-hydroxy propionylamino)-benzoïque de formule : 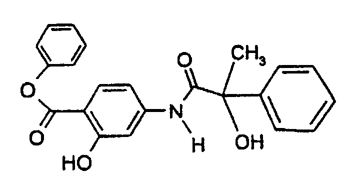
(d) l'ester méthylique de l'acide 2-méthoxy-4-(3,3,3-trifluoro-2-hydroxy-2-methyl propionylamino)-benzoïque de formule : 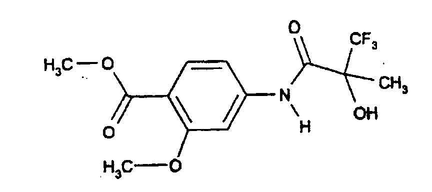
(e) l'ester phénylique de l'acide 2-chloro-4-(3,3,3-trifluoro-2-hydroxy-2-methyl propionylamino)-benzoïque de formule : 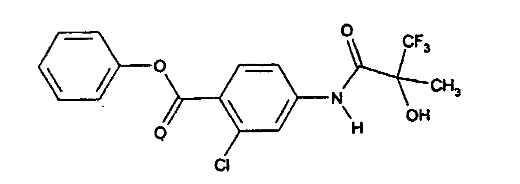
(f) l'ester méthylique de l'acide 2-ethyl-4-(3,3,3-trifluoro-2-hydroxy-2-methyl propionylamino)-benzoïque de formule : 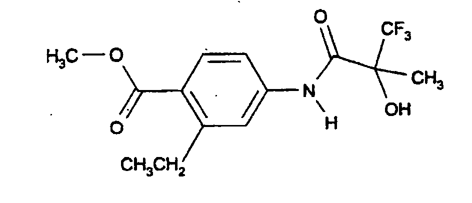
(g) l'ester 3,5-bis-trifluorométhyl-benzylique de l'acide 2-hydroxy-4-(3,3,3-trifluoro-2-hydroxy-2-methyl propionylamino)-benzoïque de formule 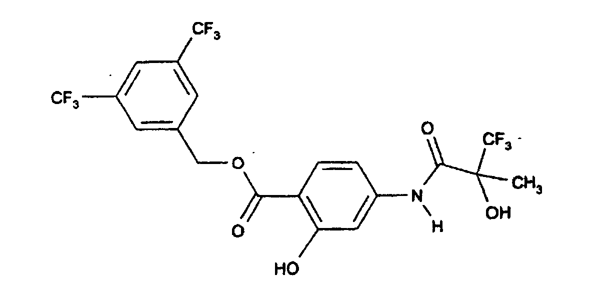
(h) l'ester tert-butylique de l'acide 2-hydroxy-4-(3,3,3-trifluoro-2-hydroxy-2-methyl propionylamino)-benzoïque de formule : 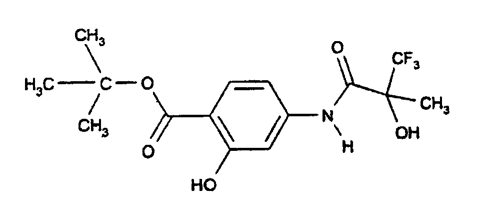
(i) l'ester 3-morpholino-propylique de l'acide 2-hydroxy-4-(3,3,3-trifluoro-2-hydroxy-2-methyl propionylamino)-benzoïque de formule : 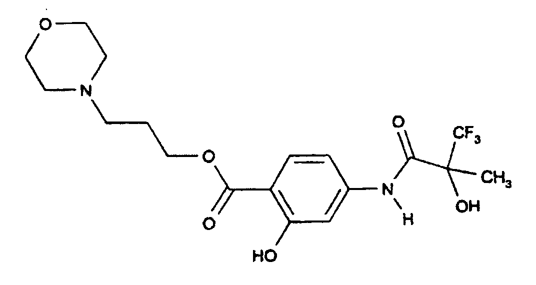
(j) l'ester 4-octyl-phénylique de l'acide 2-hydroxy-4-(3,3,3-trifluoro-2-hydroxy-2-methyl propionylamino)-benzoïque de formule : 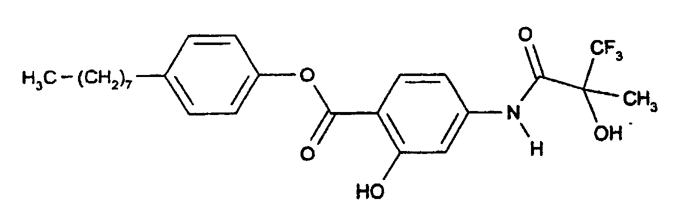
(k) l'ester 4- (O-methyloxime)-phénylique de l'acide 2-hydroxy-4-(3,3,3-trifluoro-2-hydroxy-2-methyl propionylamino)-benzoïque de formule 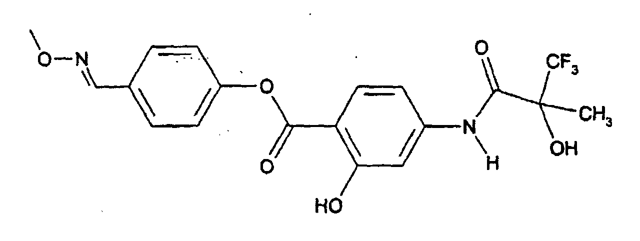
(I) l'ester méthylique de l'acide 2-hydroxy-4-(3,3,3-trifluoro-2-hydroxy-2-methyl propionylamino)-benzoïque de formule : 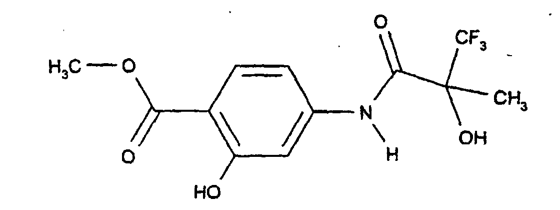 Spectre RMN: ¹H (500 MHz) dans DMSO : conforme à la structure attendue

### Exemple 3 : Lotion quotidienne

On prépare une composition comprenant les constituants suivants :
- composé de l'exemple 1 1 g
- éthanol 30 g
- parfum, colorants qs
- eau déminéralisée qsp 100 g

### Exemple 4 : Gel Liposomé

On prépare une composition comprenant les constituants suivants :
- composé de l'exemple 2(a) 0,5 g
- Carbomer 0,25 g
- triéthanolamine qs pH = 7
- conservateurs qs
- eau déminéralisée qsp 100 g

### Exemple 5 : Lotion antichute

On prépare une composition comprenant les constituants suivants :
- composé de l'exemple 2(b) 1 g
- propylène glycol 10 g
- isopropanol qsp 100 g

On applique 1 ml de cette lotion sur le cuir chevelu, à la fréquence de une à deux fois par jour.

### Exemple 6 : Lotion antichute

On prépare une composition comprenant les constituants suivants :
- composé de l'exemple 2(c) 2 g
- propylène glycol 30 g
- éthanol 40,5 g
- eau qsp 100 g

On applique cette lotion sur le cuir chevelu, une à deux fois par jour, à raison de 1 ml par application.

### Exemple 7 : Lotion antichute

On prépare une composition comprenant les constituants suivants :
- composé de l'exemple 2(d) 1 g
- monométhyléther de propylèneglycol (Dowanol PM de Dow) 20 g
- hydroxypropylcellulose (Klucel G de Herculès) 3 g
- éthanol 40 g
- eau qsp 100 g

On applique cette lotion épaissie sur le cuir chevelu, une à deux fois par jour, à raison d'un ml par application.

Avec chacune des compositions décrites dans les exemples 3 à 7 ci-dessus, on a constaté, après plusieurs mois de traitement et selon les sujets traités, un ralentissement de la chute des cheveux.

## Revendications

1. Composé répondant à la formule (I) suivante : 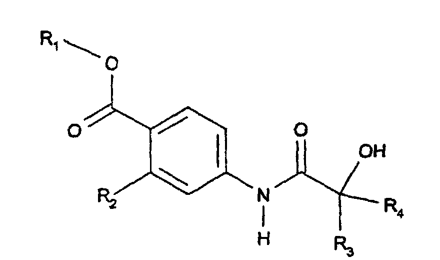 dans laquelle :
* R₁ représente :
- un groupement alkyle aliphatique en C₁-C₁₂, linéaire ou ramifié, éventuellement substitué par un ou plusieurs groupements choisis parmi OH, NH₂, SH; CONHR⁵, COOR⁵, OR⁵, SR⁵, SiOR⁵, NHR⁵ dans lesquels R⁵ est un radical alkyle linéaire ou ramifié en C₁-C₄; CN, CF₃, halogène, ou par un ou plusieurs cycles en C₃-C₆ aliphatiques ou aromatiques, éventuellement hétérocycliques;
- un groupement aryle, éventuellement substitué par un ou plusieurs groupements OH, NH₂, SH, COOH; CONHR⁶, COOR⁶, OR⁶, SR⁶, NHR⁶ dans lesquels R⁶ est un radical alkyle linéaire ou ramifié en C₁-C₁₂; CN, CF₃, halogène ou par un ou plusieurs cycles en C₃-C₆ aliphatiques ou aromatiques, éventuellement hétérocycliques; ou
- un polymère ou copolymère naturel ou synthétique portant une ou plusieurs combinaisons des groupements suivants: hydroxyle, carboxylate, amine primaire, amine secondaire, amine tertiaire, thiol, aldéhyde.
* R₂ représente un atome d'hydrogène, d'halogène, un radical CN, CF₃, OH, OCF₃, COOH, R⁷, OR⁷ ou OCOR⁷ dans lesquels R⁷ désigne un radical alkyle aliphatique en C₁-C₄ linéaire ou ramifié:
* R₃ représente un radical alkyle linéaire ou ramifié en C₁-C₄ mono- ou poly-halogéné, ou un radical aryle, éventuellement substitué par un ou plusieurs groupements choisis parmi OH, NH₂, SH, CN, CF₃, halogène, COOH, CONHR⁸, COOR⁸, OR⁸, SR⁸, NHR⁸ dans lesquels R⁸ est un radical alkyle en C₁-C₁₂, linéaire ou ramifié.
* R₄ représente un radical alkyle en C₁-C₄ linéaire ou ramifié, éventuellement substitué par un atome d'halogène, ou représente un radical CF₃.

2. Composé selon la revendication 1, dans lequel R₁ représente un groupement aryle éventuellement substitué, et notamment un groupement phényle ou benzyle.

3. Composé selon l'une des revendications précédentes, dans lequel R₂ représente un atome d'hydrogène, un atome d'halogène et notamment un atome de chlore, ou un radical hydroxy.

4. Composé selon l'une des revendications précédentes, dans lequel R₃ représente un radical alkyle halogéné, notamment fluoré, ou un radical phényle, en particulier le radical CF₃.

5. Composé selon l'une des revendications précédentes, dans lequel R₄ représente un radical méthyle.

6. Composé selon l'une des revendications précédentes, choisi parmi, seul ou en mélange :
- l'ester 4-(O-methyloxime)-phénylique de l'acide 2-hydroxy-4-(3,3,3-trifluoro-2-hydroxy-2-methyl propionylamino)-benzoïque;
- l'ester phénylique de l'acide 2-hydroxy-4-(3,3,3-trifluoro-2-hydroxy-2-methyl propionylamino)-benzoïque;
- l'ester méthylique de l'acide-4-(3,3,3-trifluoro-2-hydroxy-2-methyl propionylamino)-benzoïque;
- l'ester para-tolyl de l'acide 2-hydroxy-4-(3,3,3-trifluoro-2-hydroxy-2-methyl propionylamino)-benzoïque;
- l'ester phenylique de l'acide 2-hydroxy-4-(2-phenyl-2-hydroxy propionylamino)-benzoïque;
- l'ester méthylique de l'acide 2-méthoxy-4-(3,3,3-trifluoro-2-hydroxy-2-methyl propionylamino)-benzoïque;
- l'ester phénylique de l'acide 2-chloro-4-(3,3,3-trifluoro-2-hydroxy-2-methyl propionylamino)-benzoïque;
- l'ester méthylique de l'acide 2-ethyl-4-(3,3,3-trifluoro-2-hydroxy-2-methyl propionylamino)-benzoïque;
- l'ester 3,5-bis-trifluorométhyl-benzylique de l'acide 2-hydroxy-4-(3,3,3-trifluoro-2-hydroxy-2-methyl propionylamino)-benzoïque;
- l'ester tert-butylique de l'acide 2-hydroxy-4-(3,3,3-trifluoro-2-hydroxy-2-methyl propionylamino)-benzoïque;
- l'ester 3-morpholino-propylique de l'acide 2-hydroxy-4-(3,3,3-trifluoro-2-hydroxy-2-methyl propionylamino)-benzoïque;
- l'ester 4-octyl-phénylique de l'acide 2-hydroxy-4-(3,3,3-trifluoro-2-hydroxy-2-methyl propionylamino)-benzoïque; et
- l'ester méthylique de l'acide 2-hydroxy-4-(3,3,3-trifluoro-2-hydroxy-2-methyl propionylamino)-benzoïque.

7. Composition comprenant, dans un milieu physiologiquement acceptable, au moins un composé tel que défini dans l'une des revendications 1 à 6.

8. Composition cosmétique comprenant, dans un milieu cosmétiquement acceptable, au moins un composé tel que défini dans l'une des revendications 1 à 6.

9. Composition selon l'une des revendications 7 à 8, dans laquelle ledit composé de formule (I) est présent en une quantité comprise entre 0,0001 et 30% en poids par rapport au poids total de la composition, notamment entre 0,01 et 10% en poids, et de préférence entre 0,1 et 5% en poids.

10. Composition selon l'une des revendications 7 à 9, comprenant en outre un ou plusieurs composés améliorant l'activité sur la repousse et/ou sur le freinage de la chute des cheveux.

11. Composition selon l'une des revendications 7 à 10, se présentant sous la forme de lotion plus ou moins épaissie, de gel, d'émulsion, de crème, sous forme liposomée.

12. Utilisation d'au moins un composé répondant à la formule (I) selon l'une des revendications 1 à 6 pour la préparation d'une composition physiologiquement acceptable pour diminuer et/ou freiner la chute des cheveux et/ou des poils, voire induire et/ou stimuler leur croissance.

13. Utilisation selon la revendication 12, dans une composition cosmétique.

14. Procédé de traitement cosmétique des cheveux, des poils et/ou du cuir chevelu, dans lequel on applique sur les cheveux, les poils et/ou le cuir chevelu, une composition cosmétique telle que définie selon l'une des revendications 7 à 11, à laisser celle-ci en contact avec les cheveux et/ou le cuir chevelu et/ou les poils, et éventuellement à rincer.

## Claims

1. Compound corresponding to the following formula (I): 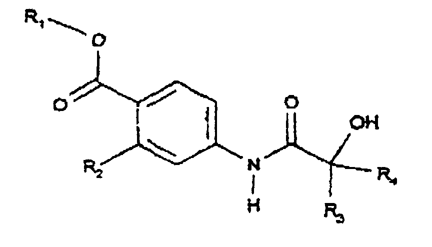 in which:
* R₁ represents:
- a linear or branched C₁-C₁₂ aliphatic alkyl group, optionally substituted with one or more groups chosen from OH, NH₂, SH; CONHR⁵, COOR⁵, OR⁵, SR⁵, SiOR⁵, NHR⁵ in which R⁵ is a linear or branched C₁-C₄ alkyl radical; CN, CF₃, halogen, or with one or more aliphatic or aromatic, optionally heterocyclic, C₃-C₆ rings;
- an aryl group, optionally substituted with one or more groups OH, NH₂, SH, COOH; CONHR⁶, COOR⁶, OR⁶, SR⁶, NHR⁶ in which R⁶ is a linear or branched C₁-C₁₂ alkyl radical; CN, CF₃, halogen, or with one or more aliphatic or aromatic, optionally heterocyclic, C₃-C₆ rings; or
- a natural or synthetic polymer or copolymer carrying one or more combinations of the following groups: hydroxyl, carboxylate, primary amine, secondary amine, tertiary amine, thiol, aldehyde,
* R₂ represents a hydrogen or halogen atom, a radical CN, CF₃, OH, OCF₃, COOH, R⁷, OR⁷ or OCOR⁷ in which R⁷ denotes a linear or branched C₁-C₄ aliphatic alkyl radical,
* R₃ represents a mono- or polyhalogenated linear or branched C₁-C₄ alkyl radical, or an aryl radical, optionally substituted with one or more groups chosen from OH, NH₂, SH, CN, CF₃, halogen, COOH, CONHR⁸, COOR⁸, OR⁸, SR⁸, NHR⁸ in which R⁸ is a linear or branched C₁-C₁₂ alkyl radical,
* R₄ represents a linear or branched C₁-C₄ alkyl radical, optionally substituted with a halogen atom, or represents a CF₃ radical.

2. Compound according to Claim 1, in which R₁ represents an optionally substituted aryl group, and in particular a phenyl or benzyl group.

3. Compound according to one of the preceding claims, in which R₂ represents a hydrogen atom, a halogen atom and in particular a chlorine atom, or a hydroxyl radical.

4. Compound according to one of the preceding claims, in which R₃ represents a halogenated, in particular fluorinated, alkyl radical or a phenyl radical, in particular the CF₃ radical.

5. Compound according to one of the preceding claims, in which R₄ represents a methyl radical.

6. Compound according to one of the preceding claims, chosen from, alone or in the form of a mixture:
- 4- (O-methyloxime)phenyl ester of 2-hydroxy-4-(3,3,3-trifluoro-2-hydroxy-2-methylpropionylamino)benzoic acid;
- phenyl ester of 2-hydroxy-4-(3,3,3-trifluoro-2-hydroxy-2-methylpropionylamino)benzoic acid;
- methyl ester of 4- (3,3,3-trifluoro-2-hydroxy-2-methylpropionylamino)benzoic acid;
- para-tolyl ester of 2-hydroxy-4-(3,3,3-trifluoro-2-hydroxy-2-methylpropionylamino)benzoic acid;
- phenyl ester of 2-hydroxy-4-(2-phenyl-2-hydroxypropionylamino)benzoic acid;
- methyl ester of 2-methoxy-4-(3,3,3-trifluoro-2-hydroxy-2-methylpropionylamino)benzoic acid;
- phenyl ester of 2-chloro-4-(3,3,3-trifluoro-2-hydroxy-2-methylpropionylamino)benzoic acid;
- methyl ester of 2-ethyl-4-(3,3,3-trifluoro-2-hydroxy-2-methylpropionylamino)benzoic acid;
- 3,5-bis(trifluoromethyl)benzyl, ester of 2-hydroxy-4-(3,3,3-trifluoro-2-hydroxy-2-methylpropionylamino)benzoic acid;
- tert-butyl ester of 2-hydroxy-4- (3,3,3-trifluoro-2-hydroxy-2-methylpropionylamino)benzoic acid;
- 3-morpholinopropyl ester of 2-hydroxy-4- (3,3,3-trifluoro-2-hydroxy-2-methylpropionylamino)benzoic acid;
- 4-octylphenyl ester of 2-hydroxy-4-(3,3,3-trifluoro-2-hydroxy-2-methylpropionylamino)benzoic acid; and
- methyl ester of 2-hydroxy-4-(3,3,3-trifluoro-2-hydroxy-2-methylpropionylamino)benzoic acid.

7. Composition comprising, in a physiologically acceptable medium, at least one compound as defined in one of Claims 1 to 6.

8. Cosmetic composition comprising, in a cosmetically acceptable medium, at least one compound as defined in one of Claims 1 to 6.

9. Composition according to either of Claims 7 and 8, in which the said compound of formula (I) is present in a quantity of between 0.0001 and 30% by weight relative to the total weight of the composition, in particular between 0.01 and 10% by weight, and preferably between 0.1 and 5% by weight.

10. Composition according to one of Claims 7 to 9, comprising, in addition, one or more compounds which enhance the activity on hair regrowth and/or on slowing down hair loss.

11. Composition according to one of Claims 7 to 10, which is provided in the form of a lotion which is thickened to a greater or lesser degree, a gel, an emulsion, a cream, in liposomal form.

12. Use of at least one compound corresponding to formula (I) according to one of Claims 1 to 6, for the preparation of a physiologically acceptable composition for reducing and/or slowing down the loss of head hair and/or body hair, or even inducing and/or stimulating its growth.

13. Use according to Claim 12, in a cosmetic composition.

14. Method for the cosmetic treatment of head hair, body hair and/or the scalp, in which a cosmetic composition as defined according to one of Claims 7 to 11 is applied to the head hair, the body hair and/or the scalp, leaving it in contact with the head hair and/or the scalp and/or the body hair, and optionally rinsing off.

## Patentansprüche

1. Verbindung der folgenden Formel (I): 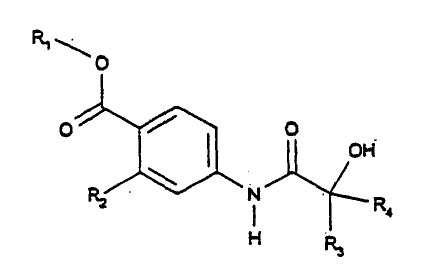 wobei in der Formel:
- R₁ bedeutet:
- eine geradkettige oder verzweigte aliphatische C₁₋₁₂-Alkylgruppe, die gegebenenfalls mit einer oder mehreren Gruppen, die unter OH, NH₂, SH, CONHR⁵, COOR⁵, OR⁵, SR⁵, SiOR⁵, NHR⁵, wobei R⁵ eine geradkettige oder verzweigte C₁₋₄-Alkylgruppe bedeutet, CN, CF₃ oder Halogen ausgewählt sind, oder mit einem oder mehreren aliphatischen oder aromatischen, gegebenenfalls heterocyclischen C₃₋₆-Ringen substituiert ist;
- eine Arylgruppe, die gegebenenfalls mit einer oder mehreren Gruppen OH, NH₂, SH, COOH, CONHR⁶, COOR⁶, OR⁶, SR⁶, NHR⁶, wobei R⁶ eine geradkettige oder verzweigte C₁₋₁₂-Alkylgruppe bedeutet, CN, CF₃ oder Halogen oder mit einem oder mehreren aliphatischen oder aromatischen, gegebenenfalls heterocyclischen C₃₋₆-Ringen substituiert ist;
- ein natürliches oder synthetisches Polymer oder Copolymer, das eine oder mehrere Kombinationen der folgenden Gruppen trägt: Hydroxy, Carboxylat, primäres Amin, sekundäres Amin, tertiäres Amin, Thiol, Aldehyd;
- R₂ Wasserstoff, Halogen, CN, CF₃, OH, OCF₃, COOH, R⁷, OR⁷ oder OCOR⁷ bedeutet, wobei R⁷ eine geradkettige oder verzweigte aliphatische C₁₋₄-Alkylgruppe ist;
- R₃ eine geradkettige oder verzweigte, mono- oder polyhalogenierte C₁₋₄-Alkylgruppe oder eine Arylgruppe bedeutet, die gegebenenfalls mit einer oder mehreren Gruppen substituiert ist, die unter OH, NH₂, SH, CN, CF₃, Halogen, COOH, CONHR⁸, COOR⁸, OR⁸, SR⁸ oder NHR⁸ ausgewählt sind, wobei R⁸ eine geradkettige oder verzweigte C₁₋₁₂-Alkylgruppe ist;
- R₄ eine geradkettige oder verzweigte, gegebenenfalls mit einem Halogenatom substituierte C₁₋₄-Alkylgruppe oder CF₃ bedeutet.

2. Verbindung nach Anspruch 1, wobei R₁ eine gegebenenfalls substituierte Arylgruppe und insbesondere Phenyl oder Benzyl bedeutet.

3. Verbindung nach einem der vorhergehenden Ansprüche, wobei R₂ Wasserstoff, Halogen und insbesondere Chlor oder Hydroxy bedeutet.

4. Verbindung nach einem der vorhergehenden Ansprüche, wobei die Gruppe R₃ eine halogenierte und insbesondere fluorierte Alkylgruppe oder eine Phenylgruppe und insbesondere CF₃ bedeutet.

5. Verbindung nach einem der vorhergehenden Ansprüche, wobei R₄ Methyl bedeutet.

6. Verbindung nach einem der vorhergehenden Ansprüche, die einzeln oder im Gemisch unter den folgenden Verbindungen ausgewählt ist:
· 2-Hydroxy-4-(3,3,3-trifluor-2-hydroxy-2-methyl-propionylamino)-benzoesäure-4-(O-methyloxim)phenyl-ester;
· 2-Hydroxy-4-(3,3,3-trifluor-2-hydroxy-2-methyl-propionylamino)-benzoesäurephenylester;
· 4-(3,3,3-Trifluor-2-hydroxy-2-methyl-propionylamino)benzoesäuremethylester;
· 2-Hydroxy-4-(3,3,3-trifluor-2-hydroxy-2-methyl-propionylamino)-benzoesäure-p-tolyl-ester;
· 2-Hydroxy-4-(2-phenyl-2-hydroxy-propionylamino)benzoesäurephenylester;
· 2-Methoxy-4-(3,3,3-trifluor-2-hydroxy-2-methyl-propionylamino)-benzoesäuremethylester;
· 2-Chlor-4-(3,3,3-trifluor-2-hydroxy-2-methyl-propionylamino)-benzoesäurephenylester;
· 2-Ethyl-4-(3,3,3-trifluor-2-hydroxy-2-methyl-propionylam.ino)-benzoesäuremethylester;
· 2-Hydroxy-4-(3,3,3-trifluor-2-hydroxy-2-methyl-propionylamino)-benzoesäure-3,5-bis-trifluormethylbenzyl-ester;
· 2-Hydroxy-4-(3,3,3-trifluor-2-hydroxy-2-methyl-propiony)amino)-benzoesäure-*t*-butyl-ester;
· 2-Hydroxy-4-(3,3,3-trifluor-2-hydroxy-2-methyl-propionylamino)-benzoesäure-3-morpholinopropyl-ester;
· 2-Hydroxy-4-(3,3,3-trifluor-2-hydroxy-2-methyl-propionylamino)-benzoesäure-4-octylphenyl-ester; und
· 2-Hydroxy-4-(3,3,3-trifluor-2-hydroxy-2-methyl-propionylamino)-benzoesäuremethylester.

7. Zusammensetzung, die in einem physiologisch akzeptablen Medium mindestens eine Verbindung nach einem der Ansprüche 1 bis 6 enthält.

8. Kosmetische Zusammensetzung, die in einem kosmetisch akzeptablen Medium mindestens eine Verbindung nach einem der Ansprüche 1 bis 6 enthält.

9. Zusammensetzung nach einem der Ansprüche 7 bis 8, in der die Verbindung der Formel (I) in einer Menge von 0,0001 bis 30 Gew.-%, bezogen auf das Gesamtgewicht der Zusammensetzung, insbesondere 0,01 bis 10 Gew.-% und vorzugsweise 0,1 bis 5 Gew.-% vorliegt.

10. Zusammensetzung nach einem der Ansprüche 7 bis 9, die ferner eine oder mehrere Verbindungen enthält, die die Wirksamkeit bezüglich des Wachstums der Haare und/oder der Verlangsarnung des Haarausfalls verbessern.

11. Zusammensetzung nach einem der Ansprüche 7 bis 10, die als mehr oder weniger dickflüssige Lotion, Gel, Emulsion, Creme oder in Form von Liposomen vorliegt.

12. Verwendung mindestens einer Verbindung der Formel (I) nach einem der Ansprüche 1 bis 6 zur Herstellung einer physiologisch akzeptablen Zusammensetzung zur Verminderung und/oder Verlangsamung des Ausfalles der Haare und/oder Körperhaare oder zur Induzierung und/oder Stimulierung des Haarwachstums.

13. Verwendung nach Anspruch 12 in einer kosmetischen Zusammensetzung.

14. Verfahren zur kosmetischen Behandlung der Haare, der Körperhaare und/oder der Kopfhaut, das darin besteht, auf das Haar, die Körperhaare und/oder die Kopfhaut eine kosmetische Zusammensetzung nach einem der Ansprüche 7 bis 11 aufzutragen, das Haar und/oder die Kopfhaut und/oder die Körperhaare mit der Zusammensetzung in Kontakt zu lassen und ggf. zu spülen.
